(19) Europäisches Patentamt
European Patent Office
Office européen des brevets

(11) **EP 1 125 913 B1**

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(51) Int Cl.⁷: **C07C 51/58**, C07C 63/68, C07C 63/70

(21) Anmeldenummer: **01101158.2**

(22) Anmeldetag: **23.01.2001**

(54) **Verfahren zur Herstellung von 3,5-Bis-(trifluormethyl)-benzoylchloriden und neue 3,5-Bis-(trihalogenomethyl)- und 3,5-Dimethylbenzoylhalogenide**

Preparation of 3,5-bis-(trifluormethyl)-benzoylchlorid and novel benzoylhalides: 3,5-bis-(trihalomethyl)- and 3,5-dimethyl-

Préparation du chlorure de 3,5-bis-(trifluorométhyle)benzoyle et nouveaux halogénures de benzoyle: 3,5-bis-(trihalogénométhyle)- et 3,5-diméthyle-

(84) Benannte Vertragsstaaten:
**AT BE CH CY DE DK ES FI FR GB GR IE IT LI LU MC NL PT SE TR**

(30) Priorität: **03.02.2000 DE 10004717**

(43) Veröffentlichungstag der Anmeldung:
**22.08.2001 Patentblatt 2001/34**

(73) Patentinhaber: **BAYER AG**
**51368 Leverkusen (DE)**

(72) Erfinder:
- **Marhold, Albrecht, Dr.**
  **51373 Leverkusen (DE)**
- **Stölting, Jörn, Dr.**
  **51061 Köln (DE)**

(56) Entgegenhaltungen:
**DE-C- 707 955** **FR-A- 820 696**
**US-A- 4 500 471**

- **H. A. SMITH ET AL.: "Physiologically Active Compounds. III. Hydrochlorides of Amino Esters of Phenylcyclohexylglycolic Acids, of Amides of Benzilic, Phenylcyclohexyl- and Dicyclohexylglycolic, and Phenylcyclohexylacetic Acids; 2-Methylthioethyl Ester Methiodides of Substituted Benzilic Acids" JOURNAL OF ORGANIC CHEMISTRY, Nr. 24, 1959, Seiten 1301-1309, XP000995199**
- **T. S. CROFT ET AL.: "Reduction of Perfluoroacyl Halides with Organosilicon Hydrides. A Direct Synthesis of Fluorine Containing Esters and Lactones" JOURNAL OF ORGANIC CHEMISTRY, Bd. 41, Nr. 13, 1976, Seiten 2256-2258, XP002164253**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von 3,5-Bis-(trifluormethyl)-benzoylchloriden aus den entsprechenden 3,5-Dimethylbenzoesäuren und dabei als Zwischenprodukte auftretende neue 3,5-Bis-(trihalogenomethyl)- und 3,5-Dimethylbenzoylhalogenide. Im Folgenden werden 3,5-Bis-(trifluormethyl)-benzoylchloride auch als BTB bezeichnet.

BTB sind Zwischenprodukte für die Herstellung von pharmazeutischen und agrochemischen Wirkstoffen sowie Photoresist-Kompositionen.

Bekannt ist die Herstellung von BTB aus dem entsprechenden Trichlormethyl-benzoylchlorid in Anwesenheit von Flussäure und eines Lewis-Säure Katalysators (siehe US 4 500 471).

Auch bekannt ist die Herstellung von BTB aus der entsprechenden 3,5-Bis-(trifluormethyl)-benzoesäure durch Chlorierung (siehe beispielsweise J. Med. Chem. 38, 3106 (1995)). Diese Säure kann man auf zweierlei Weise erhalten, indem man

a) 1-Brom-3,5-bis-(trifluormethyl)-benzol mit Magnesium oder Lithium metallisiert (siehe Bull. Soc. Chim. Fr. 1962 (587) und Chem. Ber. 129, 233 (1996)) und dann mit Kohlendioxid oder in Gegenwart eines Palladium-Katalysators mit Kohlenmonoxid und Wasser umsetzt (siehe JP-OS 09-67 297) oder

b) 3,5-Bis-(trifluormethyl)-benzol mit einem Gemisch aus Butyllithium und Kalium-t-butanolat (siehe Synlett 1990, 747) oder nur mit Butyllithium (siehe J. Organomet. Chem. 67, 321 (1974)) und abschließend mit Kohlendioxid umsetzt.

Für den technischen Maßstab sind diese Verfahren zur Herstellung von BTB wenig geeignet, weil in allen Fällen metallorganische Verbindungen hergestellt und gehandhabt werden müssen, was nur mit großem technologischem Aufwand möglich ist. Außerdem sind 3,5-Bis-(trifluormethyl)-benzol und die entsprechende 1-Bromverbindung nur aufwendig herzustellen. Hinzu kommt die Gefahr der exothermen Zersetzung von meta-trifluormethylsubstituierten Phenyl-Magnesium- und -Lithiumverbindungen, die ebenfalls großen Aufwand für eine einigermaßen sichere Beherrschung erfordert.

Weiterhin ist bekannt, dass man 3,5-Bis-(trifluormethyl)-benzoylfluoride herstellen kann, indem man 1,3,5-Tris-(trichlormethyl)-benzole mit Wasser selektiv zu 3,5-Bis-(trichlormethyl)-benzoylchloriden verseift (siehe DB-PS 705 650) und dann mit Fluorwasserstoff oder Antimontrifluorid einen vollständigen Chlor/Fluor-Austausch vornimmt (siehe DE-PS 707 955). Ob und gegebenenfalls wie man aus 3,5-Bis-(trifluormethyl)-benzoylfluoriden die entsprechenden Benzoylchloride (= BTB) erhalten kann, ist nicht bekannt.

Es besteht deshalb ein Bedürfnis nach einem ohne besonderen Aufwand, ausgehend von einfacher zugänglichen Ausgangsprodukten sicher im technischen Maßstab durchzuführenden Verfahren zur Herstellung von BTB.

Es wurde nun ein Verfahren zur Herstellung von 3,5-Bis-(trifluormethyl)-benzoylchloriden der Formel (I) gefunden

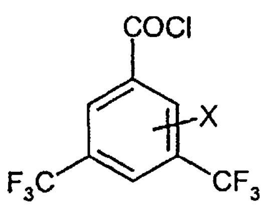

(I),

in der

X für Wasserstoff, Fluor oder Chlor steht,

das dadurch gekennzeichnet ist, dass man 3,5-Dimethylbenzoesäuren der Formel (V)

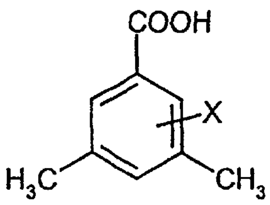

(V),

in der

X die bei Formel (I) angegebene Bedeutung hat,

in die entsprechenden Säurechloride der Formel (IV) umwandelt

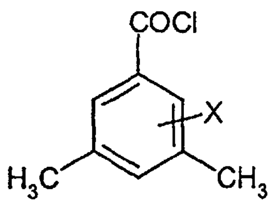

(IV),

in der

X die bei Formel (I) angegebene Bedeutung hat,

diese in den Seitenketten vollständig radikalisch chloriert und so 3,5-Bis-(trichlormethyl)-benzoylchloride der Formel (III) erhält

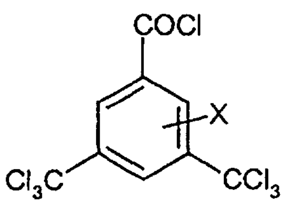

(III),

in der

X die bei Formel (I) angegebene Bedeutung hat,

diese mit wasserfreiem Fluorwasserstoff und/oder Antimonpentafluorid fluoriert und so 3,5-Bis-(trifluormethyl)-benzoylfluoride der Formel (II) erhält

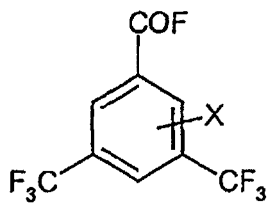

(II),

in der

X die bei Formel (I) angegebene Bedeutung hat,

und abschließend daraus durch Umsetzung mit Siliciumtetrachlorid in Gegenwart einer weiteren Lewis-Säure die entsprechende Verbindung der Formel (I) erhält.

In den Formeln (I) bis (V) steht X vorzugsweise für Wasserstoff.

Die erste Stufe des erfindungsgemäßen Verfahrens, die Herstellung der Säurechloride der Formel (IV) aus den Benzoesäuren (V) kann analog bekannten Verfahren zur Herstellung von Carbonsäurechloriden aus Carbonsäuren durchgeführt werden. Eine Möglichkeit der Umsetzung von 3,5-Dimethylbenzoesäure mit Phosphorpentachlorid ist aus Can. J. Chem. 41, 2962 (1963) eine andere mit Thionylchlorid aus J. Org. Chem. 24, 1301 (1959) bekannt. Diese Umsetzungen können für Verbindungen, in denen X für Fluor oder Chlor steht, analog durchgeführt werden.

Die zur Durchführung der ersten Stufe benötigten Benzoesäuren der Formel (V) können nach bekannten Verfahren oder analog dazu hergestellt werden. 3,5-Dimethylbenzoesäure ist kommerziell erhältlich.

Die Umwandlung zu den Säurehalogeniden der Formel (IV) kann mit Chlorierreagenzien, beispielsweise mit Thionylchlorid, Phosphortrichlorid, Phosphorpentachlorid, Phosphoroxychlorid, Oxalylchlorid oder Phosgen durchgeführt werden. Bevorzugt werden Thionylchlorid oder Oxalylchlorid eingesetzt, deren Reaktionsprodukte (Chlorwasserstoff und Schwefeldioxid bzw. Chlorwasserstoff, Kohlenmonoxid und Kohlendioxid) leicht flüchtig sind und deshalb gut entfernt werden können.

Die Umwandlung zu den Säurechloriden der Formel (IV) wird vorzugsweise in Gegenwart eines Verdünnungsmittels durchgeführt. Hierfür kommen inerte organische Lösungsmittel oder Mischungen daraus in Betracht. Beispielhaft seien genannt: aliphatische, alicyclische und aromatische Kohlenwasserstoffe wie Petrolether, Hexan, Heptan, Cyclohexan, Methylcyclohexan, Benzol, Toluol, Xylole und Decalin, halogenierte Kohlenwasserstoffe wie Chlorbenzol, Dichlorbenzole, Methylenchlorid, Chloroform, Tetrachlormethan, Dichlorethane, Trichlorethan und Tetrachlorethylen, Ether wie Diethylether, Tetrahydrofuran, 1,2-Dimethoxyethan, 1,2-Diethoxyethan, Diethylenglykoldimethylether und Anisol, Ester wie Methyl-, Ethyl- und Butylacetat und Sulfone wie Sulfolan. Pro Mol Benzoesäure der Formel (V) kann man z.B. 50 bis 150 ml Verdünnungsmittel einsetzen.

Es ist vorteilhaft, einen Überschuss des Chlorierungsreagenzes einzusetzen, beispielsweise pro Mol der Benzoesäure der Formel (V) 1,1 bis 10 Mol, bevorzugt 1,2 bis 3 Mol Chlorierreagenz.

Die Reaktionstemperatur für diese Stufe kann in einem größeren Bereich variiert werden. Sie kann beispielsweise zwischen 0 und 150°C, bevorzugt zwischen 20 und 120°C betragen.

Die Aufarbeitung nach der Reaktion kann beispielsweise durch Destillation erfolgen. Bei Verwendung der bevorzugten Chlorierungsreagenzien kann man einfach deren Überschuss und das gegebenenfalls vorhandene Verdünnungsmittel abdestillieren und den Destillationsrückstand als Rohprodukt in die nächste Stufe einsetzen.

Die zweite Stufe des erfindungsgemäßen Verfahrens, die Seitenkettenchlorierung der 3,5-Dimethylbenzoylchloride der Formel (IV) ist neu. Diese Seitenkettenchlorierung wird als radikalische Reaktion durchgeführt. Dies kann man durch erhöhte Temperatur, Bestrahlung mit einer Lichtquelle und/oder Zusatz eines Radikalstarters erreichen. Als Lichtquelle eignen sich z.B. Glühlampen, vorzugsweise Halogenlampen und Mittel- und Hochdruck-Quecksilberdampflampen. Als Radikalstarter kommen z.B. Benzoylperoxid, Di-tert.-butylperoxid, 2,2-Aza-bis-(isobuttersäurenitril) und 2-Phenylazo-2,4-dimethyl-4-methoxyvaleronitril in Frage. Bevorzugt setzt man eine Lichtquelle bei erhöhter Temperatur ein. Die Reaktionstemperatur kann z.B. zwischen 80 und 250°C, vorzugsweise 100 bis 220°C, besonders bevorzugt zwischen 110 und 190°C liegen. Dabei ist es vorteilhaft, die Chlorierung bei relativ niedrigen Temperaturen, beispielsweise 80 bis 140°C, zu beginnen und bei relativ höheren Temperaturen, beispielsweise 160 bis 250°C, zu Ende zu führen.

Als Chlorierungsmittel in dieser Stufe verwendet man im allgemeinen elementares Chlor.

Pro Mol Dimethylbenzoylchlorid der Formel (IV) kann man z.B. 6,3 bis 18 Mol, vorzugsweise 7,2 bis 12 Mol Chlorgas einsetzen.

Zur Aufarbeitung nach der Reaktion kann man gegebenenfalls vorhandenes überschüssiges Chlor verdrängen, z.B. durch Einleiten eines Inertgases wie Stickstoff oder Anlegen von Vakuum. So erhältliches Rohprodukt kann

direkt in die nächste Reaktionsstufe eingesetzt werden, wenn gewünscht kann es aber auch z.B. durch Vakuumdestillation gereinigt werden.

Die dritte Stufe des erfindungsgemäße Verfahrens ist die Fluorierung der 3,5-Bis-(trichlormethyl)-benzoylchloride der Formel (III) zu den 3,5-Bis-(trifluormethyl)-benzoylfluoriden der Formel (II). Eine Möglichkeit der Herstellung des 3,5-Bis-(trifluormethyl)-benzoylfluorids ist bereits aus DE-PS 707 955 bekannt und kann auf die Verbindungen, in denen X für Fluor oder Chlor steht, analog übertragen werden.

Die Fluorierung wird mit wasserfreier Flusssäure und/oder mit Antimonpentafluorid durchgeführt. Man kann gegebenenfalls Katalysatoren zusetzen, z.B. Lewis-Säuren wie Titantetrachlorid, Bortrichlorid oder Antimonpentafluorid, was i.a. die Reaktion beschleunigt. Bevorzugt setzt man wasserfreien Fluorwasserstoff in Mischung mit Titantetrachlorid ein. Man kann die Lewis-Säuren auch erst nach Beginn der Reaktion zusetzen.

Pro Mol Benzoylchlorid der Formel (III) kann man z.B. 7,7 bis 21 Mol (entsprechend 10 bis 200% Überschuss) wasserfreien Fluorwasserstoff oder die entsprechende Menge Antimonpentafluorid und z.B. 0 bis 0,2 Mol Lewis-Säuren einsetzen.

Die Fluorierung kann man beispielsweise so durchführen, dass man bei einer Temperatur unterhalb des Siedepunktes (bei Normaldruck) von Fluorwasserstoff beginnt, beispielsweise bei -20 bis +15°C, und zur Vervollständigung der Reaktion bei höheren Temperaturen, beispielsweise bei 100 bis 180°C, zu Ende führt. Durch den Dampfdruck des Fluorwasserstoffs können dabei Drucke bis zu 100 bar auftreten, was die Verwendung entsprechend druckfester Reaktionsgefäße bedingt. Den freiwerdenden Chlorwasserstoff entspannt man beispielsweise bei Temperaturen über +20°C über ein Druckhalteventil.

Die Aufarbeitung des nach der Fluorierung vorliegenden Reaktionsgemisches kann z.B. durch fraktionierte Destillation erfolgen.

Die abschließende vierte Stufe des erfindungsgemäßen Verfahren ist der für diese Verbindungen bisher nicht bekannt gewordene Chlor/Fluor-Austausch an der Carbonylgruppe. Diese wird mit Siliciumtetrachlorid als Reagenz in Gegenwart von einer weiteren Lewis-Säure, beispielsweise Aluminiumchlorid, Bortrifluorid, Titantetrachlorid, Eisentrichlorid oder Mischungen davon, durchgeführt.

Pro Mol Benzoylfluorid der Formel (II) kann man z.B. 0,25 bis 1 Mol (1 bis 4 Äquivalente), bevorzugt 0,3 bis 0,5 Mol Siliciumtetrachlorid und 0,01 bis 0,1 Mol, bevorzugt 0,02 bis 0,05 Mol weitere Lewis-Säure einsetzen.

Dieser Chlor/Fluor-Austausch kann beispielsweise bei Temperaturen zwischen 0 und 70°C, bevorzugt zwischen 20 und 50°C durchgeführt werden. Man kann dabei so vorgehen, die weitere Lewis-Säure entweder mit dem Benzoylfluorid der Formel (II) oder mit dem Siliciumtetrachlorid vorzulegen und die jeweils andere Komponente zuzudosieren. So kann man die Gasentwicklung gut kontrollieren.

Die Aufarbeitung des nach dem Chlor/Fluor-Austausch vorliegenden Reaktionsgemisches kann man z.B. so durchführen, dass man zunächst die festen Bestandteile abtrennt, z.B. durch Filtration, vorzugsweise nach Zusatz eines Filterhilfsmittels wie Cellulose oder ein Zeolith. Durch fraktionierte Vakuum-Destillation des Filtrats kann man das hergestellte BTB in reiner Form erhalten. Zur Desaktivierung von Resten des Siliciumtetrachlorid und/oder der weiteren Lewis-Säure kann es vorteilhaft sein, dem zu destillierenden Gemisch eine kleine Menge eines Aryl- oder Alkylphosphins zuzugeben, beispielsweise 0,1 bis 1 Gew.-%. Hierfür ist beispielsweise Triphenylphosphin geeignet.

Mit dem erfindungsgemäßen Verfahren lassen sich BTB der Formel (I) aus den gut zugänglichen 3,5-Dimethylbenzoesäuren der Formel (V) in einem technisch gut und einfach durchzuführenden Verfahren in guten Ausbeuten herstellen. Über alle Reaktionsstufen betrachtet liegt die Ausbeute deutlich höher als 60 % d.Th.

Die Verbindungen der Formeln (I) bis (IV) sind teilweise neu. Die vorliegende Erfindung betrifft deshalb auch 3,5-Bis-(trifluormethyl)-benzoylchloride der Formel (Ia)

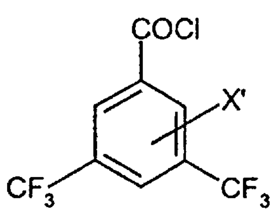

(Ia),

in der

X' für Fluor oder Chlor steht,

3,5-Bis-(trifluormethyl)-benzoylfluoride der Formel (IIa)

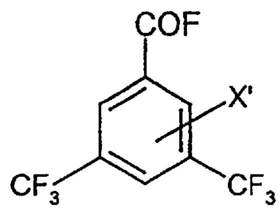

(IIa),

in der

X' für Fluor oder Chlor steht,

3,5-Bis-(trichlormethyl)-benzoylchloride der Formel (IIIa)

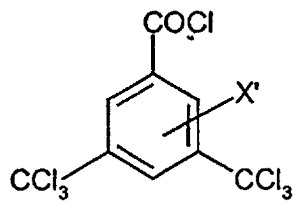

(IIIa),

in der

X' für Fluor oder Chlor steht und

3,5-Dimethylbenzoylchloride der Formel (IVa)

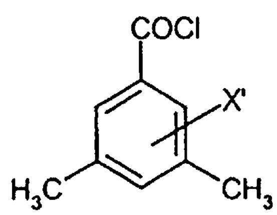

(IVa),

in der

X' für Fluor oder Chlor steht.

Die Herstellung von Verbindungen der Formeln (Ia) bis (IVa) ist oben beschrieben. Es handelt sich bei ihnen um neue Zwischenprodukte zur vorteilhaften Herstellung von 3,5-Bis-(trifluormethyl)-benzoylchlorid nach dem erfindungsgemäßen Verfahren.

## Beispiele

### Beispiel 1

3,5-Dimethylbenzoylchlorid

In einem 4 1-Planschliffreaktionsgefäß wurden 1000 g 3,5-Dimethylbenzoesäure in 450 ml Toluol vorgelegt und unter Rühren bei 60°C 80 ml Thionylchlorid im Verlaufe von 2 Stunden zugetropft, wobei eine Gasentwicklung

stattfand. Anschließend wurde zum Sieden erhitzt (102°C Innentemperatur) und 2 Stunden unter Rückfluss gekocht. Danach wurde im Verlaufe von 1,5 Stunden überschüssiges Thionylchlorid und ein Teil des Toluols bis zu einer Kopftemperatur von 102°C bei Normaldruck abdestilliert. Man ließ auf 80°C erkalten und destillierte jetzt bei 20 mbar das Toluol ab. Restmengen wurden durch Andestillation an einer Kolonne bei 20 mbar bis zum Siedepunkt von 110°C (im Sumpf) entfernt. Als Rückstand wurden 1092 g (96,7 % d.Th.) 3,5-Dimethylbenzoylchlorid erhalten.

**Beispiel 2**

3,5-Bis-(trichlormethyl)-benzoylchlorid

In einem mit einer luftgekühlten UV-Tauchlampe ausgestatteten Reaktionsgefäß wurden bei 120°C 1092 g 3,5-Dimethylbenzoylchlorid vorgelegt und 61 Stunden lang unter UV-Bestrahlung und gleichmäßiger Temperaturerhöhung auf bis zu 180°C insgesamt 4340 g Chlor eingeleitet. Nach GC-Analyse war das Edukt dann zu 100 % umgesetzt. Nach Ausblasen des Chlorüberschusses mit Stickstoff hinterblieben 2390 g (98,1 % d.Th.) 3,5-Bis-(trichlormethyl)-benzoylchlorid.

**Beispiel 3**

3,5-Bis-(trifluormethyl)-benzoylfluorid

In einem 5 l-Edelstahl-Rührautoklaven mit aufsteigendem Kühler (betrieben mit einem Kühlmittel mit einer Temperatur von -10°C) und Druckregler wurden 990 ml wasserfreie Flusssäure vorgelegt. Dann tropfte man in 30 Minuten bei -5 bis 0°C 1126 g 3,5-Bis-(trichlormethyl)-benzoylchlorid zu, wobei nur eine schwache Entwicklung von Chlorwasserstoffgas auftrat. Die Temperatur wurde auf +20°C ansteigen gelassen. Nach Beendigung der leichten Gasentwicklung (nach 1,5 Stunden) wurden in 40 Minuten 68 g Titantetrachlorid hinzugegeben. Nach Abklingen der erneuten Gasentwicklung (nach 2 Stunden) wurde die Apparatur verschlossen, 10 bar Stickstoff aufgedrückt und stufenweise auf 140°C erhitzt, wobei der entstehende Chlorwasserstoff laufend bei 25 bar entspannt wurde. Nach 11 Stunden bei 140°C war die Reaktion beendet. Der Autoklav wurde abgekühlt, entspannt, der überschüssige Fluorwasserstoff bei Normaldruck abdestilliert (248 g) und der Rückstand im Vakuum (70 bis 12 mbar) über eine Brücke destilliert (maximale Kopftemperatur: 84°C bei 12 mbar). Es blieben 43 g eines harzigen Rückstandes zurück. Das Rohdestillat wurde im Vakuum über eine 60 cm-Füllkörperkolonne mit Wilson-Spiralen fraktioniert destilliert (50 mbar, 140°C Badtemperatur, 75°C Kopftemperatur). Es wurden neben 105 g Destillationsrückstand (nicht vollkommen fluorierte Produkte, die erneut eingesetzt werden können) 633 g (81 % d.Th.) 3,5-Bis-(trifluormethyl)-benzoyifluorid mit einer Reinheit von 99,9 % (GC, Flächen-%) erhalten.

**Beispiel 4**

3,5-Bis-(trifluormethyl)-benzoylchlorid

1040 g 3,5-Bis-(trifluormethyl)-benzoylfluorid und 24 g Aluminiumchlorid wurden vorgelegt und auf 40°C erwärmt. Unter Rühren wurden in 3 Stunden 224 Siliciumtetrachlorid zugetropft, wobei die Temperatur kontrolliert wurde und 45°C nicht überstieg. Es wurde nachgerührt bis die Gasentwicklung beendet war (2 Stunden). Anschließend wurden 30 g Zeolith X133 zugegeben und abfiltriert. Erhalten wurden 1000 g eines trüben Filtrats, das mit 5 g Triphenylphosphin versetzt und bei 12 mbar über eine 70 cm-Kolonne destilliert wurde. Es wurden 623 g (83,6 % d.Th.) bei 68°C (Badtemperatur: 80°C) übergehendes 3,5-Bis-(trifluormethyl)-benzoylchlorid erhalten.

**Beispiel 5**

In einer Rührapparatur mit Gaseinleitung und Ableitung zu einem Vemichter wurden 100 g (0,593 Mol.) 3,5-Dimethylbenzoylchlorid zusammen mit 0,5 g Eisen-III-chlorid vorgelegt und bei 22-28°C innerhalb 3 Stunden 42 g Chlor eingeleitet. Durch fraktionierte Destillation wurden 78 g 2-Chlor-3,5-dimethyl-benzoylchlorid erhalten. Siedebereich: 113-114°C bei 5 mbar.

**Beispiel 6**

In einer Chlorierungsapparatur mit einer UV-Bestrahlungslampe wurden 78 g 2-Chlor-3,5-dimethylbenzoylchlorid in 100 ml trockenem 4-Chlorbenzotrifluorid vorgelegt und 1 g Phosphortrichlorid und 0,5 g Kaliumchlorid zugefügt. Die Temperatur wurde anfänglich auf 100°C eingestellt und langsam Chlor eingeleitet. Im Verlauf der Chlorierung

wurde die Innentemperatur auf Rückfluß des Lösungsmittel gesteigert. Nach 20 Stunden waren 200 g Chlor eingeleitet, und die Reaktionsmischung wurde fraktioniert destilliert. Im Siedebereich von 145-147°C bei 0,2 mbar gingen 119 g 2-Chlor-3,5-bistrichlormethyl-benzoylchlorid über. $n_D^{20}$: 1,6025

**Beispiel 7**

In einem Autoklaven aus Edelstahl wurden 100 ml HF und 2 ml Antimonpentachorid eingefüllt. Bei 0°C wurde eine Lösung von 119 g 2-Chlor-3,5-bistrifluormethylbenzoylchlorid in 50ml trockenen Dichlormethan zudosiert. Anschließend wurden 10 bar Stickstoff aufgedrückt und danach die Temperatur stufenweise bis auf 145°C erhöht. Der entstehende Chlorwasserstoff wurde über einen auf -15°C gekühlten Rückflußkühler bei 25 bar entspannt. Nach 9 Stunden war die Entwicklung von Chlorwasserstoff beendet, und es wurde auf Raumtemperatur abgekühlt. Danach wurde noch vorhandener Fluorwasserstoff und Dichlormethan abdestilliert. Das Reaktionsprodukt wurde einer Feindestillation unterworfen. Im Siedebereich 76-77°C bei 13 mbar gingen 68 g 2-Chlor-3,5-bistrifluormethyl-benzoylfluorid über. $n_D^{20}$: 1,4292.

**Patentansprüche**

1. Verfahren zur Herstellung von 3,5-Bis-(trifluormethyl)-benzoylchloriden der Formel (I)

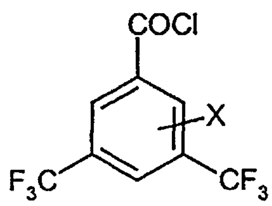

(I),

in der

X für Wasserstoff, Fluor oder Chlor steht,

**dadurch gekennzeichnet, dass** man 3,5-Dimethylbenzoesäuren der Formel (V)

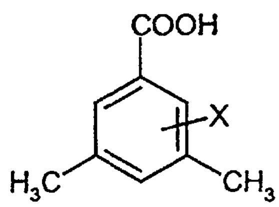

(V),

in der

X die bei Formel (I) angegebene Bedeutung hat,

in die entsprechenden Säurechloride der Formel (IV) umwandelt

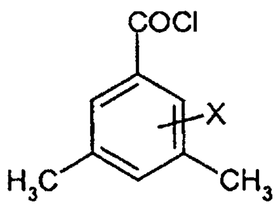

(IV),

in der

X die bei Formel (I) angegebene Bedeutung hat,

diese in den Seitenketten vollständig radikalisch chloriert und so 3,5-Bis-(trichlormethyl)-benzoylchloride der Formel (III) erhält

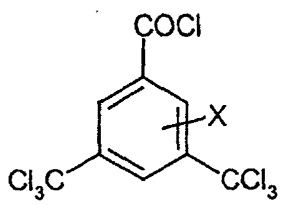

(III),

in der

X die bei Formel (I) angegebene Bedeutung hat,

diese mit wasserfreiem Fluorwasserstoff und/oder Antimonpentafluorid fluoriert und so 3,5-Bis-(trifluormethyl)-benzoylfluoride der Formel (II) erhält

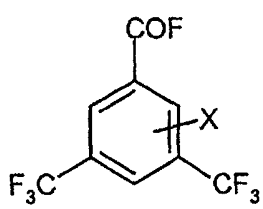

(II),

in der

X die bei Formel (I) angegebene Bedeutung hat,

und abschließend daraus durch Umsetzung mit Siliciumtetrachlorid in Gegenwart einer weiteren Lewis-Säure die entsprechende Verbindung der Formel (I) erhält.

**2.** Verfahren nach Anspruch 1, **dadurch gekennzeichnet, dass** man die Umwandlung zu den Säurechloriden der Formel (IV) mit einem Chlorierungsreagenz aus der Gruppe Thionylchlorid, Phosphortrichlorid, Phosphorpentachlorid, Phosphoroxychlorid, Oxalylchlorid und Phosgen und in Gegenwart eines Verdünnungsmittels durchführt.

**3.** Verfahren nach Anspruch 2, **dadurch gekennzeichnet, dass** man pro Mol der Benzoesäure der Formel (V) 1,1 bis 10 Mol Chlorierungsreagenz umsetzt und bei 0 bis 150°C arbeitet.

**4.** Verfahren nach Ansprüchen 1 bis 3, **dadurch gekennzeichnet, dass** man die radikalische Seitenkettenchlorierung bei erhöhter Temperatur, Bestrahlung mit einer Lichtquelle und/oder einem Zusatz eines Radikalstarters bei 80 bis 250°C und mit elementarem Chlor durchführt.

5. Verfahren nach Anspruch 4, **dadurch gekennzeichnet, dass** man 7,2 bis 12 Mol Chlorgas pro Mol Dimethylbenzoylchlorid der Formel (IV) einsetzt.

6. Verfahren nach Ansprüchen 1 bis 5, **dadurch gekennzeichnet, dass** man die Fluorierung mit wasserfreier Flusssäure unter Zusatz einer Lewis-Säure durchführt, wobei man pro Mol Benzoylchlorid der Formel (III) 7,7 bis 21 Mol wasserfreien Fluorwasserstoff einsetzt.

7. Verfahren nach Ansprüchen 1 bis 6, **dadurch gekennzeichnet, dass** man die Umsetzung mit Siliciumtetrachlorid in Gegenwart von Aluminiumchlorid, Bortrifluorid, Titantetrachlorid, Eisentrichlorid oder Mischungen davon durchführt.

8. Verfahren nach Ansprüchen 1 bis 7, **dadurch gekennzeichnet, dass** man die Umsetzung mit Siliciumtetrachlorid mit 0,25 bis 1 Mol Siliciumtetrachlorid und 0,01 bis 0,1 Mol weiterer Lewis-Säure, jeweils bezogen auf 1 Mol Benzoylfluorid der Formel (II), durchführt.

9. 3,5-Bis-(trifluormethyl)-benzoylchloride der Formel (Ia)

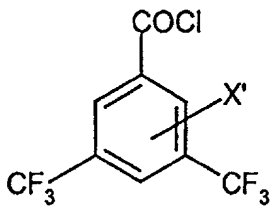

(Ia),

in der

X' für Fluor oder Chlor steht.

10. 3,5-Bis-(trifluormethyl)-benzoylfluoride der Formel (IIa)

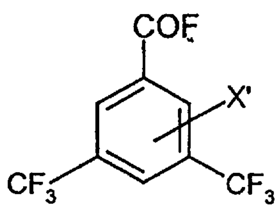

(IIa),

in der

X' für Fluor oder Chlor steht.

11. 3,5-Bis-(trichlormethyl)-benzoylchloride der Formel (IIIa)

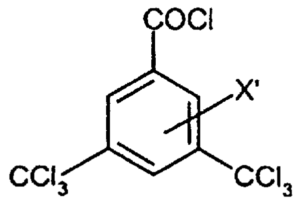

(IIIa),

in der

X' für Fluor oder Chlor steht.

**12.** 3,5-Dimethylbenzoylchloride der Formel (IVa)

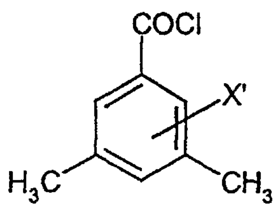

(IVa),

in der

X' für Fluor oder Chlor steht.

**Claims**

**1.** Process for the preparation of 3,5-bis-(trifluoromethyl)-benzoyl chlorides of the formula (I)

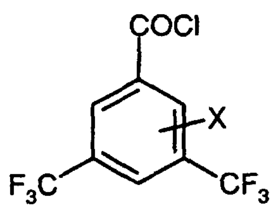

(I),

in which
X is hydrogen, fluorine or chlorine,
**characterized in that** 3,5-dimethylbenzoic acids of the formula (V)

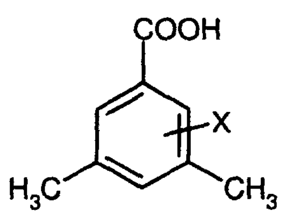

(V),

in which
X has the meaning given in the case of formula (I),
are converted into the corresponding acid chlorides of the formula (IV)

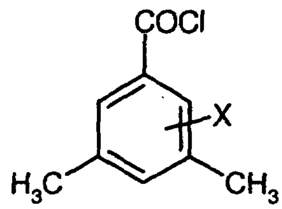

(IV),

in which
X has the meaning given in the case of formula (I),
said acid chlorides are completely free-radically chlorinated in the side chains, giving 3,5-bis-(trichloromethyl)-benzoyl chlorides of the formula (III)

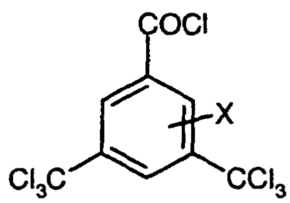

(III),

in which
X has the meaning given in the case of formula (I),
which are fluorinated with anhydrous hydrogen fluoride and/or antimony pentafluoride, giving 3,5-bis-(trifluoromethyl)-benzoyl fluorides of the formula (II)

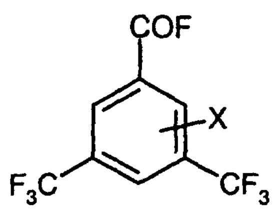

(II),

in which
X has the meaning given in the case of formula (I),
and then the corresponding compound of the formula (I) is obtained therefrom by reaction with silicon tetrachloride in the presence of a further Lewis acid.

2. Process according to Claim 1, **characterized in that** the conversion to the acid chlorides of the formula (IV) is carried out with a chlorinating reagent from the group thionyl chloride, phosphorus trichloride, phosphorus pentachloride, phosphorus oxychloride, oxalyl chloride and phosgene, and in the presence of the diluent.

3. Process according to Claim 2, **characterized in that**, per mole of the benzoic acid of the formula (V), 1.1 to 10 mol of chlorinating reagent are reacted, and the operating temperature is 0 to 150°C.

4. Process according to Claims 1 to 3, **characterized in that** the free-radical side-chain chlorination is carried out at elevated temperature, with irradiation by a light source and/or the addition of a free-radical initiator at 80 to 250°C and with elemental chlorine.

5. Process according to Claim 4, **characterized in that** 7.2 to 12 mol of chlorine gas are used per mole of dimethylbenzoyl chloride of the formula (IV).

**6.** Process according to Claims 1 to 5, **characterized in that** the fluorination is carried out with anhydrous hydrofluoric acid with the addition of a Lewis acid, where, per mole of benzoyl chloride of the formula (III), 7.7 to 21 mol of anhydrous hydrogen fluoride are used.

**7.** Process according to Claims 1 to 6, **characterized in that** the reaction with silicon tetrachloride is carried out in the presence of aluminium chloride, boron trifluoride, titanium tetrachloride, iron trichloride or mixtures thereof.

**8.** Process according to Claims 1 to 7, **characterized in that** the reaction with silicon tetrachloride is carried out with 0.25 to 1 mol of silicon tetrachloride and 0.01 to 0.1 mol of further Lewis acid, in each case based on 1 mol of benzoyl fluoride of the formula (II).

**9.** 3,5-Bis-(trifluoromethyl)-benzoyl chlorides of the formula (Ia)

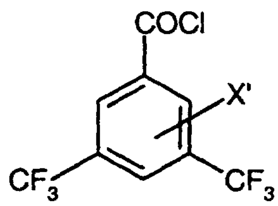

(Ia),

in which
X' is fluorine or chlorine.

**10.** 3,5-Bis-(trifluoromethyl)-benzoyl fluorides of the formula (IIa)

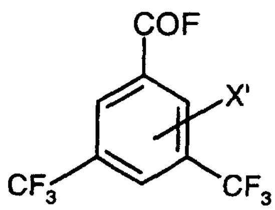

(IIa),

in which
X' is fluorine or chlorine.

**11.** 3,5-Bis-(trichloromethyl)-benzoyl chlorides of the formula (IIIa)

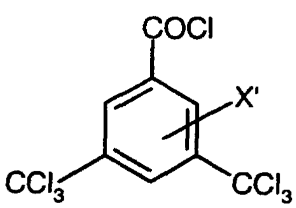

(IIIa),

in which
X' is fluorine or chlorine.

**12.** 3,5-Dimethylbenzoyl chlorides of the formula (IVa)

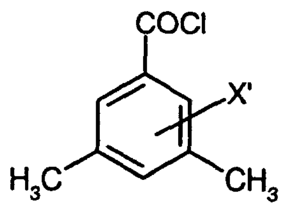

(IVa),

in which
X' is fluorine or chlorine.

**Revendications**

**1.** Procédé de préparation de chlorures de 3,5-bis-(trifluorométhyl)benzoyle de la formule (I) :

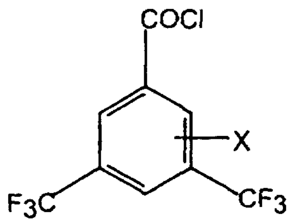

(I)

dans laquelle :

X représente l'atome d'hydrogène, de fluor ou de chlore,

**caractérisé en ce que** l'on convertit l'acide 3,5-diméthylbenzoïque de la formule (V) :

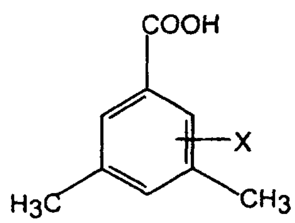

(V)

dans laquelle :

X a la signification indiquée pour la formule (I),

en le chlorure d'acide correspondant de 1a formule (IV) :

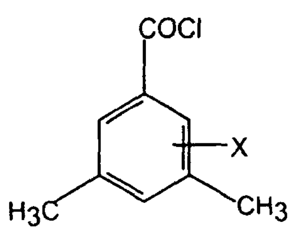

(IV)

dans laquelle :

X a la signification indiquée pour la formule (I),

celui-ci est complètement chloré de manière radicalaire au niveau des chaines latérales et l'on obtient le chlorure de 3,5-bis-(trichlorométhyl)-benzoyle de la formule (III) :

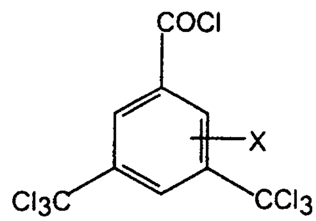

(III)

dans laquelle :

X a la signification indiquée pour la formule (I),

celui-ci est fluoré avec du fluorure d'hydrogène anhydre et/ou du pentafluorure d'antimoine et l'on obtient le fluorure de 3,5-bis-(trifluorométhyl)benzoyle de la formule (II) :

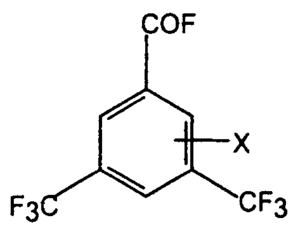

(II)

dans laquelle :

X a la signification indiquée pour la formule (I),

et ensuite, on obtient par réaction avec le tétrachlorure de silicium en présence d'un autre acide de Lewis, le composé approprié de la formule (I).

2. Procédé suivant la revendication 1, **caractérisé en ce que** l'on réalise la conversion en le chlorure d'acide de formule (IV) avec un réactif de chloration choisi parmi le groupe consistant en le chlorure de thionyle, le trichlorure de phosphore, le pentachlorure de phosphore, l'oxychlorure de phosphore, le chlorure d'oxalyle et le phosgène, et en présence d'un agent de dilution.

3. Procédé suivant la revendication 2, **caractérisé en ce que** l'on fait réagir, par mole de l'acide benzoïque de la formule (V), 1,1 à 10 moles du réactif de chloration et on travaille de 0 à 150°C.

4. Procédé suivant les revendications 1 à 3, **caractérisé en ce que** l'on réalise la chloration radicalaire des chaines latérales , à température élevée, par irradiation avec une source lumineuse et/ou par addition d'un initiateur de radicaux de 80 à 250°C et avec du chlore élémentaire.

5. Procédé suivant la revendication 4, **caractérisé en ce que** l'on met en oeuvre, 7,2 à 12 moles de chlore gazeux par mole du chlorure de diméthylbenzoyle de la formule (IV).

6. Procédé suivant les revendications 1 à 5, **caractérisé en ce que** l'on réalise la fluoration avec de l'acide fluorhydrique anhydre avec addition d'un acide de Lewis, où l'on met en oeuvre par mole du chlorure de benzoyle de la formule (III), 7,7 à 21 moles de fluorure d'hydrogène anhydre.

**7.** Procédé suivant les revendications 1 à 6, **caractérisé en ce que** l'on réalise la réaction avec le tétrachlorure de silicium en présence de chlorure d'aluminium, de trifluorure de bore, de tétrachlorure de titane, de trichlorure de fer ou de leurs mélanges.

**8.** Procédé suivant les revendications 1 à 7, **caractérisé en ce que** l'on réalise la réaction avec le tétrachlorure de silicium avec 0,25 à 1 mole de tétrachlorure de silicium et 0,01 à 0,1 mole d'un autre acide de Lewis, chaque fois sur base de 1 mole du fluorure de benzoyle de la formule (II).

**9.** Chlorures de 3,5-bis(trifluorométhyl) benzoyle de la formule (Ia) :

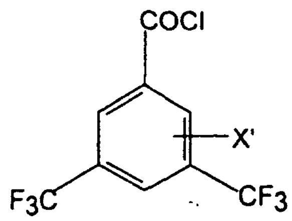

(Ia)

dans laquelle :

X' représente l'atome de fluor ou de chlore.

**10.** Fluorures de 3,5-bis(trifluorométhyl)-benzoyle de la formule (IIa) :

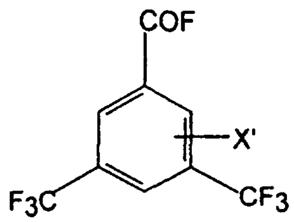

(IIa)

dans laquelle :

X' représente l'atome de fluor ou de chlore.

**11.** Chlorures de 3,5-bis(trichlorométhyl)-benzoyle de la formule (IIIa) :

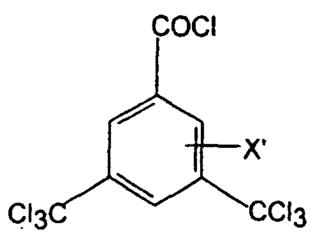

(IIIa)

dans laquelle :

X' représente l'atome de fluor ou de chlore.

**12.** Chlorures de 3,5-diméthyl) benzoyle de la formule (IVa) :

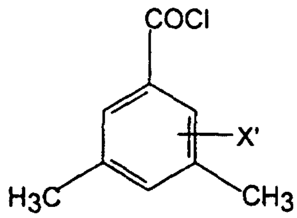

(VIa)

dans laquelle :

X' représente l'atome de fluor ou de chlore.